# EUROPEAN PATENT SPECIFICATION

(11) **EP 3 914 164 B1**
(45) Date of publication and mention of the grant of the patent: **26.11.2025**
(21) Application number: 20744372.2
(22) Date of filing: 21.01.2020
(51) Int. Cl.: A61B 17/00, A61B 17/11, A61B 1/018, A61F 2/04, A61B 90/30, A61F 5/00, A61B 90/00

(54) **SINGLE ANASTOMOSIS GASTROINTESTINAL TRACT BYPASS ENDOSCOPIC SYSTEMS**
EINZELANASTOMOSE MAGEN-DARM-BYPASS ENDOSKOPISCHE SYSTEME
SYSTÈMES ENDOSCOPIQUES DE PONTAGE DU TRACTUS GASTRO-INTESTINAL À ANASTOMOSE UNIQUE

(30) Priority: 22.01.2019 US 201962795230 P
(43) Date of publication of application: 01.12.2021
(73) Proprietor: Mayo Foundation for Medical Education and Research, Rochester, Minnesota 55905 (US)
(72) Inventor: ABU DAYYEH, Barham K., Rochester, Minnesota 55902 (US)
(74) Representative: Fish & Richardson P.C.
(86) International application number: PCT/US2020/014374
(87) International publication number: WO 2020/154269

(56) References cited:
- CA-A1- 2 997 727
- US-A1- 2006 020 247
- US-A1- 2006 036 267
- US-A1- 2006 189 845
- US-A1- 2008 243 031
- US-A1- 2012 253 259
- US-A1- 2012 253 259
- US-A1- 2019 298 401
- US-B2- 7 803 195

## Description

### CROSS-REFERENCE TO RELATED APPLICATIONS

This application claims the benefit of U.S. Provisional Application Serial No. 62/795,230, filed January 22, 2019.

### BACKGROUND

### 1. Technical Field

This document relates to devices for the medical treatment of conditions such as obesity and metabolic diseases. For example, this document relates to devices for bypassing portions of the gastrointestinal tract to reduce weight and/or to improve diabetes control.

### 2. Background Information

Obesity is a global problem crossing age, ethnic, and socioeconomic boundaries. In general, obesity means having too much body fat. Morbid obesity is a serious health condition that can interfere with basic physical functions such as breathing or walking. Individuals who are morbidly obese are at greater risk for illnesses including diabetes, high blood pressure, sleep apnea, gastroesophageal reflux disease, infertility, low back pain, asthma, gallstones, osteoarthritis, heart disease, and cancer. Billions of dollars are spent each year treating millions of individuals around the world suffering from such diseases. Many people suffering from morbid obesity find it nearly impossible to lose weight by controlling their diet and exercising.

While obesity and metabolic disease have reached pandemic proportions, yet to date non-surgical treatment modalities focusing on life-style interventions or pharmacotherapies have failed to achieve sufficient weight loss or have weight independent impact on the metabolic consequences of obesity. Bariatric surgery has not only offered a select group of morbidly obese patients effective and durable weight loss, but has also ushered a better understanding of the role of gastrointestinal tract in regulating energy intake and metabolism through weight-loss dependent and independent pathways coning the term metabolic surgery that is applicable to metabolic disease, such as type II diabetes, independent from weight. It is now clear the bariatric surgery results in significant weight loss through the manipulation of multiple gastric and small intestinal pathways, yet the majority of its weight independent metabolic effect results from the bypass of the first 150 cm of the small intestines to allow mixing of pancreaticobiliary secretions with food in the distal jejunum. Given the poor penetrance and patient acceptance of bariatric and metabolic surgery, minimally invasive surgical and endoscopic options with improved safety, efficacy, and patient acceptance have been under intense development efforts.

The single Anastomosis Gastric Bypass, also known as "mini-gastric bypass" is a minimally invasive procedure performed with laparoscopic technique, where the surgeon first reduces the size of the "working" stomach by separating a tube-like pouch of stomach from the rest of the stomach. This tubular gastric pouch is then connected (anastomosed) to the intestine, bypassing up to 150 cm of the upper part of the intestine. This technique differs from the traditional Roux-en-Y Bypass (RYGB) which requires two connections (anastomoses), thus improved patient acceptance and safety. This technique is very effective for both weight loss and diabetes resolution, with typical weight loss ranging between 30% to 40 % of body weight. Long-term data shows that this procedure results in higher weight loss and better resolution of diabetes than the traditional RYGB. The superior diabetes remission rate of this procedure is attributed to both the greater weight loss and the longer pancreaticobiliary limb length. Despite improved safety this is still an invasive surgery with poor patient acceptance and penetrance, and bile reflux through the single gastrojejunal anastomosis causing gastritis and esophagitis has been a major limitation of this approach.

US 2012/253259 A1 describes a modular system treating metabolic disorders comprising an anchoring element, a tubular implant adapted for placement within the gastro-intestinal tract, and a docking feature.

CA 2 997 727 A1 describes an intragastric device configured for deployment in a stomach of a person comprising a catheter, a first and second wire mesh structure that are flexibly coupled by a connection.

### SUMMARY

The present invention is defined by the independent claim 1. The dependent claims depict additional embodiments of the invention.

This document provides devices and methods for the endoscopic treatment of conditions such as obesity and metabolic diseases. For example, this document provides devices and methods for bypassing portions of the gastrointestinal (GI) tract which can result in decreased nutritional uptake, weight loss, and improvement in diabetes control. The devices and methods described herein can be endoscopically-installed, and include a creation of a single anastomosis and bypass of the stomach. This endoscopic technique will address the elements of the bile reflux problem completely. To achieve this, the technique includes the use of a single endoscopic accessory that allows the retraction of the jejunum (about 150cm in some cases) using a balloon over-tube. The techniques can also include the use of an illuminated snare or a small caliber endoscope connected to a light source coming from the gastric channel of the over-tube that can be used as a target to create a puncture between the stomach and jejunum. A needle catheter and wire can be used to make the puncture and thereafter introducing a wire loop that will be used to advance an approximating stent mounted on a dilation catheter.

In one aspect, this disclosure is directed to a device that is implantable in a gastrointestinal (GI) tract of a patient for reducing caloric uptake and weight of the patient. The device includes a first stent comprising a first self-expanding framework and a first covering attached to the first self-expanding framework. The device also includes a second stent comprising a second self-expanding framework and a second covering attached to the second self-expanding framework. The first self-expanding framework, when expanded, includes: (i) a proximal flange; (ii) a distal flange; and (iii) a waist portion extending between the proximal and distal flanges. The first stent defines a first lumen extending between the proximal flange and the distal flange. The second self-expanding framework, when expanded, includes: (a) a distal end portion that is coupleable, *in situ,* with the first stent such that the second stent extends from the proximal flange, the distal end portion defining a distal opening; (b) a proximal end portion defining a proximal opening; (c) a tubular portion extending between the distal end portion and the proximal end portion; and (d) a collar extending radially outward from the proximal end portion of the tubular portion. The second stent defines a second lumen extending between the proximal opening and the distal opening. The second lumen is in fluid communication with the first lumen while the second stent is coupled with the first stent. The first stent and the second stent can be coupled using magnetism. The second stent can comprise a material that assumes a flexible and malleable state when heated that can conform to the shape of the first stent such that the first stent and the second stent are coupled when the material cools to body temperature. The second stent can be installed such that the distal end of the second stent is within the first stent, such that the first stent and the second stent are coupled when the second stent self-expands within the first stent. The collar can include a self-expanding covered member. The material that assumes a flexible and malleable state when heated can be a thermoplastic material. The collar can engage with the cardia of the stomach.

Such a device may optionally include one or more of the following features. In some embodiments, the device also includes a tubular member comprising a flexible tube that is coupleable, *in situ,* with the first stent such that the flexible tube extends from the distal flange. The tubular member defines a third lumen. The third lumen is in fluid communication with the first lumen and the second lumen while the second stent and the flexible tube are coupled with the first stent.

In another aspect, this disclosure is directed to a system for deploying an implantable device. The system includes an implantable device and a snare device. The snare device is slidably disposable in the third lumen such that a distal end portion of the snare device can be inserted into the third lumen at the proximal end portion of the flexible tube and extended out of the lateral opening of the flexible tube. The snare device includes: a sheath defining a fourth lumen and an elongate flexible snare member slidably disposed within the fourth lumen and a distal end portion of the snare member configured to emit illumination during use within the GI tract.

Particular embodiments of the subject matter described in this document can be implemented to realize one or more of the following advantages. In some embodiments, the devices and methods provided herein can cause weight loss and improvement in diabetes by, among other potential mechanisms, reducing the caloric intake and absorption of an individual. The methods for implanting the devices can be performed endoscopically, thus avoiding the need for the more invasive open or laparoscopic surgical procedures. The endoscopic technique for implanting the devices can provide total direct visualization and stabilization of the GI tract anatomy in which the devices are implanted and can bypass longer segments of the gastrointestinal tract given utilization of deep enteroscopy techniques and distal release of the device. In some embodiments, each of the proximal and distal ends of the device are anchored in relation to the GI tract to definitively position the device and to provide effective migration resistance. In some embodiments, such anchoring of each of the proximal and distal ends of the device is performed during deployment either sequentially or substantially simultaneously to further enhance the definitive positioning of the device. The devices described herein are also advantageously design to prevent or inhibit reflux of stomach acid and bile.

Unless otherwise defined, all technical and scientific terms used herein have the same meaning as commonly understood by one of ordinary skill in the art to which this invention pertains. Although methods and materials similar or equivalent to those described herein can be used to practice the invention, suitable methods and materials are described below. In case of conflict between the present specification and a reference mentioned herein, the present specification, including definitions, will control. In addition, the materials, methods, and examples are illustrative only and not intended to be limiting.

The details of one or more embodiments of the invention are set forth in the accompanying drawings and the description below. Other features, objects, and advantages of the invention will be apparent from the description and drawings, and from the claims.

### DESCRIPTION OF THE DRAWINGS

FIG. 1 illustrates a first step of an example GI tract bypass procedure comprising positioning an enteroscope and over-tube in a GI tract including a stomach and portion of the small intestines.
FIG. 2 illustrates another step of the example GI tract bypass procedure.
FIG. 3 illustrates another step of the example GI tract bypass procedure.
FIG. 4a illustrates another step of the example GI tract bypass procedure.
FIGs. 4b and 4c illustrate enlarged views of a portion of FIG. 4a.
FIG. 5 illustrates another step of the example GI tract bypass procedure.
FIG. 6 illustrates another step of the example GI tract bypass procedure.
FIG. 7 illustrates another step of the example GI tract bypass procedure.
FIG. 8 illustrates another step of the example GI tract bypass procedure.
FIG. 9 illustrates another step of the example GI tract bypass procedure.
FIG. 10 illustrates an optional step of the example GI tract bypass procedure.

Like reference numbers represent corresponding parts throughout.

### DETAILED DESCRIPTION

This document provides devices and methods for the medical treatment of conditions such as obesity and various metabolic diseases. For example, this document provides devices and methods for bypassing portions of the GI tract that can reduce nutritional uptake, decrease weight, and improve diabetes control.

The devices and methods described herein can be endoscopically-installed, and include a creation of a single anastomosis. This endoscopic technique will address the elements of the bile reflux problem completely. To achieve this, the technique includes the use of a single endoscopic accessory that allows the retraction of the jejunum (about 150cm in some cases) using a balloon over-tube. The techniques can also include the use of an illuminated snare or small caliber endoscope with a light source coming from the gastric channel of the over-tube. The overtube and enteroscope will approximate and stabilize a loop or distal duodenum or jejunum to the stomach. Light from an illuminated snare or small caliber endoscope can be used as a target to create a puncture between the stomach and jejunum using a needle advanced through the working channel of the enteroscope in the small intestines. A loop wire can be introduced through the needle and thereafter used as a wire loop to advance an approximating stent mounted on a dilation catheter.

Once the wire is placed, a lumen opposing fully-covered nitinol (e.g., 20-30mm) self-expanding metal stent with its proximal gastric phalange incorporated into a soft polymer or silicon covering or bumper with an incorporated supporting metal ring that in some iteration can be magnetized to lock and support a second gastric bypassing stent, and a distal jejunal phalange having a flat a-traumatic circular configuration within the jejunum is placed. The whole sent is constraint over a soft tapered dilating catheter, that in some iterations can have a conducting cautery tip that can be used to introduce a cutting current to aid in the delivery system passage from the stomach to the duodenum or jejunum, with a loop that allows the delivery system to be tied to the placed loop wire and pulled to create a lumen opposing anastomosis. The whole lumen opposing stenting apparatus is pulled over a dilating pull-though catheter similar to a PEG tube.

Once across the desired anastomosis (e.g., gastrojejunostomy), the lumen opposing stent is deployed and expand from a constrained configuration by pulling a constraining wire or sheath that expands the stent and releases the pull through delivery catheter that in some iterations can be pulled through the overtube channel accommodating the enteroscope, once the lumen opposing stent has been deployed. Once the first stent is placed across the gastrojejunal ("GJ") or gastroduodenal ("GD") anastomosis, a second fully-covered lumen opposing nitinol stent of a larger fanning diameter (e.g., 25-35 mm) with a bypassing polymer liner sleeve liner (e.g., 40-60cm) welded on its locking distal phalange is deployed over a wire within the first locking stent. The distal liner serves dual purposes of bypassing an additional portion (e.g., about 40-60 cm) of small intestines and as anti-bile reflux mechanism. In some embodiments, the proximal phalange of the locking second lumen opposing stent has a metal ring that in some iteration can be magnetized to lock and support the two stents. In some iterations, the proximal phalange of the second lumen opposing stent terminates in the stomach creating a duodenal / jejunal bypass. In other embodiments, the second lumen opposing metal stent is part of an esophageal anchoring system that also bypasses the stomach, and in some embodiments is based on a soft polymer cylindrical portion that lies within the esophagus and a nitinol covered circular anti-torque and anti-tilting anchoring portion within the cardia. The locking system and straight configuration of the stents allows for a stable anti-migration and anti-reflux mechanism that bypasses the stomach, duodenum, and part of the jejunum.

In another iteration of this technology, a GJ anastomosis is created once the jejunum is approximated to the stomach, as described above using the retracting over tube. Then, an anastomosis needle rather that a stent can be used to approximate the structures. The anastomosis needle can be comprised of a polymer circular hollow tube maintained in straight configuration via a needle inner catheter. A suture is also running through the hollow tube to allow it to be cinched and locked in a circular format once deployed. Once the needle advances to the targeted organ with the circular tube over it (in straight configuration), the needle is withdrawn allowing the soft tube to take a circular configuration in the targeted organ (e.g., stomach or jejunum) that needs to be approximated for endoscopic anastomosis creation. The withdrawal of the inner needle catheter allows the soft tube to take a circular configuration within the targeted organ (in this case the jejunum) and returns the locking suture back to the structure to be anastomosed to (in this case the stomach). A cinching device cinches the two structures together (stomach and jejunum), locks the circular tube in the jejunum, and cuts the locking suture in the stomach. The system fixes the two structures together (stomach and jejunum) and is an anti-migration mechanism for the bypass stenting system described above. This needle can be delivered through the over-tube described above or via endoscopic ultrasound as the needle system will be compatible with linear endoscopic ultrasound scope for use in other indications such as gallbladder drainage, anti-reflux therapy.

In yet another iteration of this technology, a GJ anastomosis is created once the jejunum is approximated to the stomach as described above using the over-tube described above and a lumen opposition sent is placed in the same technique as above, however, rather than bypassing the stomach with a locking stents, a needle is used to occlude the pylorus. In some embodiments, it is an endoscopic ultrasound needle system that delivers a series of 3 to 5 t-tags in sequence. The t-tags can be interconnected with a proline suture. Once the t-tags are deployed in the targeted structure via EUS (e.g., duodenum via pylorus) by activating a release system that pulls on an inner guiding catheter to release these interconnected t-tags sequentially and pulls the suture to the entry structure to be fixed to. This device can be used in lieu of the gastric bypassing stent to close the pylorus and allow gastric content to bypass the duodenum and drain thought the created gastrojejunostomy, without a prosthesis that bypasses the stomach.

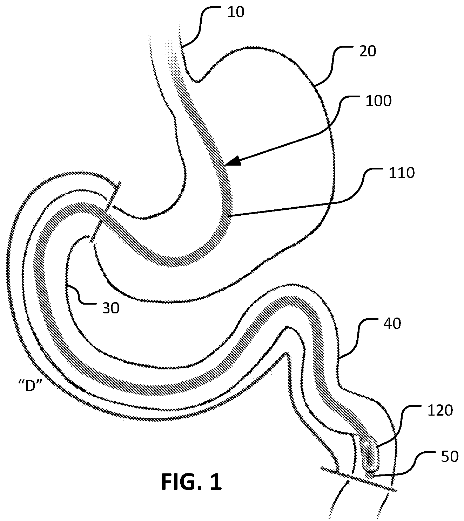

Referring to **FIG. 1****,** an example over-tube 100 can be positioned over an enteroscope 50 and the combination can be advanced through a patient's esophagus 10, stomach 20, duodenum 30, and into a portion of a jejunum 40. The over-tube 100 can include a flexible tube 110 defining a first lumen extending longitudinally there through that is configured to slidably receive the enteroscope 50. The wall of the flexible tube 110 can also define at least a second lumen and a third lumen. The third lumen can extend from a proximal end portion of the flexible tube 110 to a lateral opening defined in a sidewall of the flexible tube 110, as described further below. An inflatable member 120 is attached to a distal end portion of the flexible tube 110. The inflatable member 120 is in fluid communication with the second lumen of the flexible tube 110 such that pressurizing the second lumen causes the inflatable member 120 to radially expand/inflate.

The distance "D" is the distance between the pylorus of the stomach 20 and the portion of the jejunum 40 to be anastomosed to the stomach 20 (as described further below). In some embodiments, the distance D is between 140 cm and 160 cm, or between 120 cm and 180 cm, or between 100 cm and 180 cm, or between 80 cm and 200 cm, or between 60 cm and 220 cm. The clinician operator advances the over-tube 100 and enteroscope 50 to position the inflatable member 120 at the distance D at the portion of the jejunum 40 to be anastomosed to the stomach 20. Direct visualization (using the enteroscope 50) and/or fluoroscopy can be used to advance the inflatable member 120 to the target position at the portion of the jejunum 40 to be anastomosed to the stomach 20.

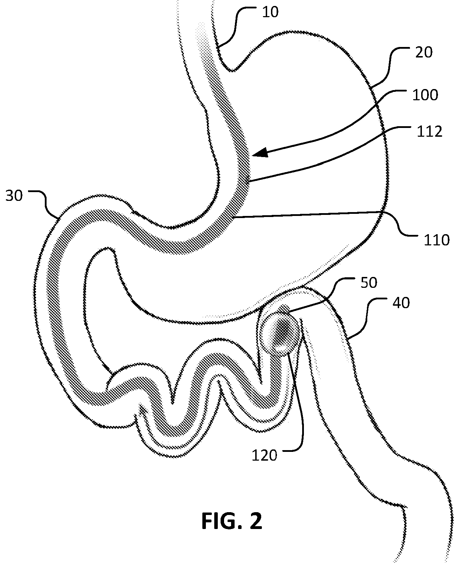

Referring also to **FIG. 2****,** when the inflatable member 120 is positioned at the distance D, the clinician can then expand/inflate the inflatable member 120 by pressurizing the second lumen of the flexible tube 110. In some embodiments, saline is used as the inflation media. Other fluids, such as air or nitrogen, can alternatively be used as the inflation media. When the inflatable member 120 is expanded, the inflatable member 120 becomes releasably anchored to the inner wall of the portion of the jejunum 40 to be anastomosed to the stomach 20.

While the expanded inflatable member 120 is anchored to the inner wall of the portion of the jejunum 40, the clinician can then pull back the over-tube 100 and enteroscope 50. In doing so, the portion of the jejunum 40 to be anastomosed to the stomach 20 can become abutted against the stomach 20 (as depicted in FIG. 2). A visualization modality such as fluoroscopy can be used to ascertain whether the portion of the jejunum 40 to be anastomosed to the stomach 20 is abutted against the stomach 20 as desired.

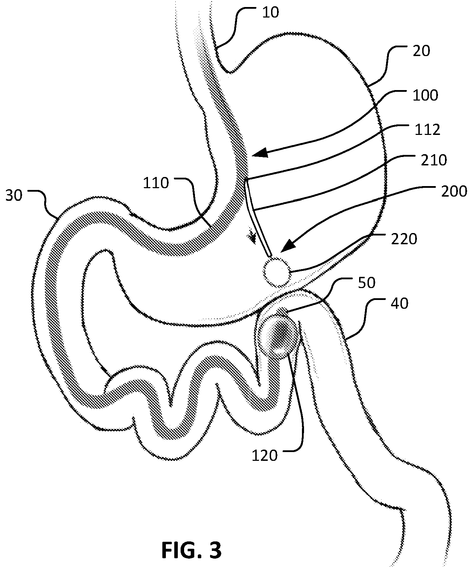

Referring to **FIG. 3****,** while the portion of the jejunum 40 to be anastomosed to the stomach 20 is abutted against the stomach 20, a snare device 200 that is slidably disposed in the third lumen defined by the wall of the flexible tube 110 can be extended into the stomach 20 as shown. The snare device 200 is extended so that it exits from the third lumen of the flexible tube 110 at a lateral opening 112 defined through the wall of the flexible tube 110. In some iterations this lumen can accommodate a small caliber gastroscope with a light source.

The snare device 200 includes a sheath 210 defining a fourth lumen. The snare device 200 also includes an elongate flexible snare member 220 that is slidably disposed within the fourth lumen of the sheath 210.

At least a distal end portion of the snare member 220 is configured to emit illumination during use. For example, in some cases the snare member 220 (or one or more portions thereof) comprises a fiber optic member that can be illuminated using an external light source, such as an LED. Accordingly, while the snare member 220 is illuminated, the enteroscope 50 can be used to directly visualize the illuminated snare member 200 (through the walls of the jejunum 40 and the stomach 20). When the illuminated snare member 220 is visualized via the enteroscope 50, the clinician can ascertain that the snare member 220 is in the desired position for the next step of the procedure.

In some embodiments, the flexible tube 110 includes additional features to facilitate the positioning of the snare device 200 in the desired position for the next step of the procedure. For example, in some embodiments the flexible tube 110 (or a portion of the flexible tube 110 that includes the lateral opening 112) is controllably rotatable about its longitudinal axis. Then, by rotating the flexible tube 110 (or the rotatable portion of the flexible tube 110) the lateral opening 112 can be positioned in an orientation such that the snare device 200 and its snare member 220 will emerge and be positioned in the desired position for the next step of the procedure.

In some embodiments, the wall of the flexible tube 110 includes multiple lumens that terminate in a lateral opening (i.e., multiple lumens like the third lumen of the flexible tube 110 that terminates at the lateral opening 112). Then, the snare device 200 and its snare member 220 can be inserted and caused to emerge in the desired position for the next step of the procedure by the selection of the most suitably positioned lumen and lateral opening. In some embodiments, the flexible tube 110 can be both controllably rotatable about its longitudinal axis and can have multiple lumens that terminate in a lateral opening (i.e., multiple lumens like the third lumen of the flexible tube 110 that terminates at the lateral opening 112).

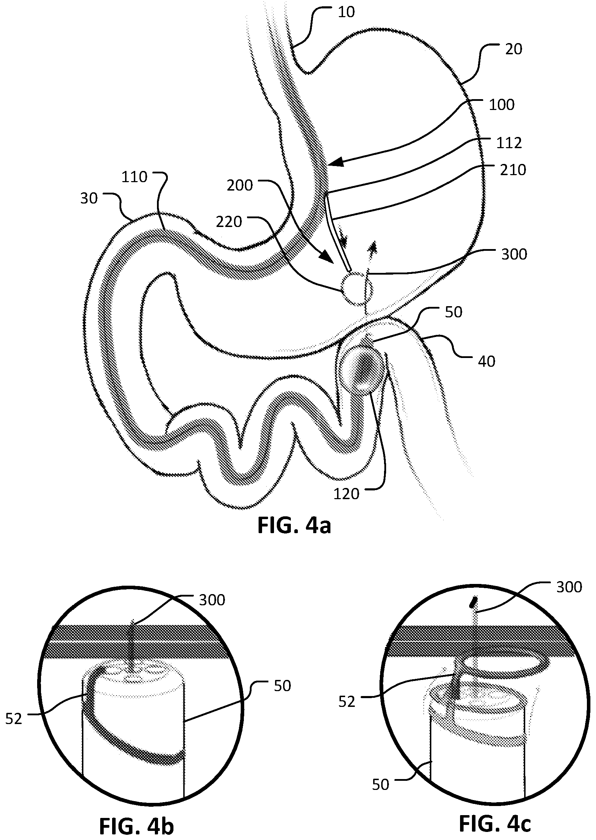

Referring also to **FIGs. 4a-4c****,** while the snare member 220 is in the desired position within the stomach 20, a wire 300 can be extended from the distal end of the flexible tube 110 or of the enteroscope 50. The looped wire 300 will be introduced through needle that punctures the walls of the jejunum 40 and the stomach 20. In some embodiments, a needle is used to create the punctures and the wire 300 is extended through the sheath of the needle.

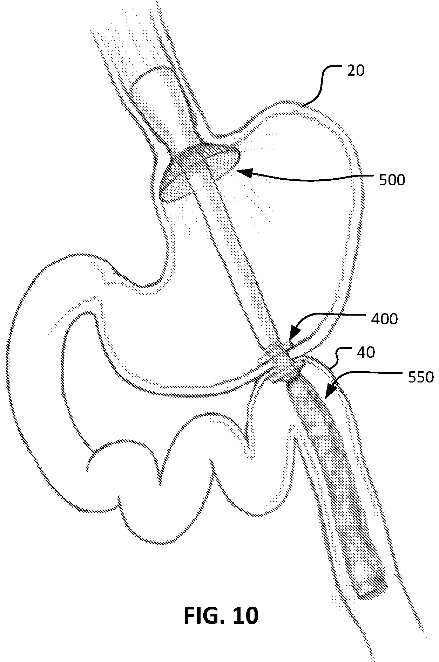

As depicted in FIGs. 4b and 4c, in some embodiments a stretchy flexible pyloric occlusion loop device 310 (or simply "loop 310") can be pre-installed on the outer diameter of a distal end portion of the enteroscope 50 (or on the outer diameter of a distal end portion of the flexible tube 110 if the wire 300 is being deployed through the flexible tube 110). The loop 310 can be advanced or pushed using its shaft 52 so that the loop 310 disengages from the outer diameter of the enteroscope 50 (as shown in FIG. 4c). Accordingly, the wire 300 will be extending through the loop 310 (as shown in FIG. 4c). The loop 310 can be used later to engaged it with the anastomosis stent device 400 in the jejunum 40. Thereafter, the loop 310 (while engaged with the anastomosis stent device 400 in the jejunum 40) can be used to assist in the deployment and engagement of a tubular member 550 comprising a flexible tube that is coupleable, *in situ,* with the anastomosis stent device 400 (as described further below in reference to FIG. 10).

As the wire 300 is advanced farther, the wire 300 will pass within the loop defined by the snare member 220. The snare member 220 can then be tightened on the wire 300 (e.g., by advancing the sheath 210, or by pulling back on the snare member 220 until the snare member 220 is mostly inside of the sheath 210).

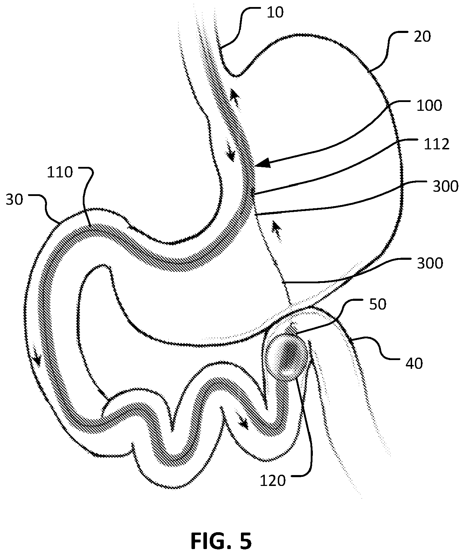

Referring also to **FIG. 5****,** when the wire 300 is coupled with the snare member 220, the clinician can pull back the snare member 220 until the wire 300 emerges from the proximal end of the third lumen of the flexible tube 110. In that configuration, the clinician will be advantageously in control of both ends of the wire 300.

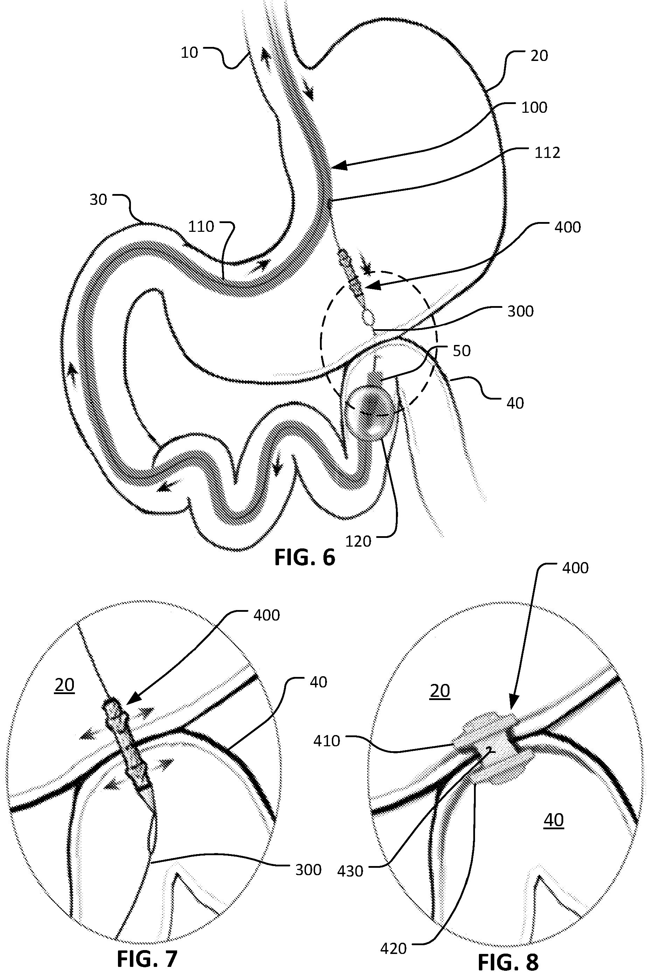

Referring to **FIG. 6****,** a clinician can use the wire 300 (with a loop extending outside the patient mouth and the other end through the working channel of the enteroscope) to advance a anastomosis stent device 400 through the third lumen of the flexible tube 110, through the lateral opening 112 defined through the wall of the flexible tube 110, and into the stomach 20 as depicted. The anastomosis stent device 400 can comprise a self-expanding framework (e.g., made of Nitinol) and a first covering (e.g., made of silicon, PTFE, ePTFE, etc.) attached to the first self-expanding framework. The distal end portion of the anastomosis stent device 400 can include a conical dilator member or electrocautery.

Referring also to **FIG. 7****,** the clinician can continue to advance the anastomosis stent device 400 so that it penetrates through the walls of the stomach 20 and the jejunum 40 (via the penetrations previously created through which the wire 300 extended.

Referring also to **FIG. 8****,** while the anastomosis stent device 400 is positioned partially in the stomach 20 and partially in the jejunum 40 (as shown in FIG. 7), the clinician can allow the anastomosis stent device 400 to self-expand to the depicted configuration. In some embodiments, the clinician can allow the expansion of the anastomosis stent device 400 by removing (e.g., "unzipping") a diametrically-constraining jacket on the anastomosis stent device 400. The expanded anastomosis stent device 400 includes a proximal flange 410, a distal flange 420, and a waist portion 430 extending between the proximal flange 410 and the distal flange 420. The anastomosis stent device 400 defines a lumen extending between the proximal flange 410 and the distal flange 420. In this fashion, an anastomosis is created between the stomach 20 and the jejunum 40 using the anastomosis stent device 400.

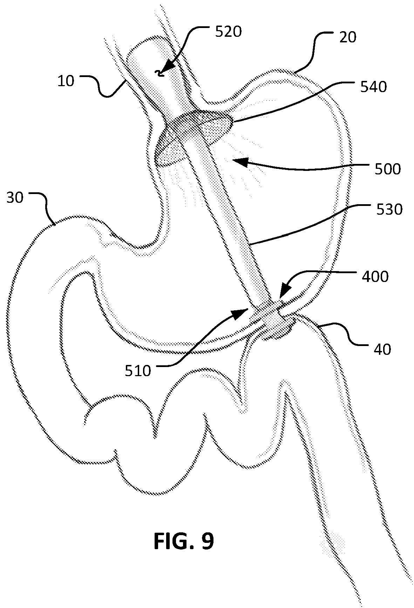

Referring to **FIG. 9****,** with the anastomosis stent device 400 implanted to create the anastomosis between the stomach 20 and the jejunum 40, the over-tube 100 and the enteroscope 50 can then be removed from the patient. Then, in some embodiments a gastric bypass stent 500 (in a diametrically-constrained configuration within a sheath that is not shown) can be installed (while on an endoscope that is not shown) such that a distal end of the gastric bypass stent 500 is within the anastomosis stent device 400. Then, in some embodiments the sheath can be pulled back so that the gastric bypass stent 500 self-expands to the configuration shown and becomes coupled with the anastomosis stent device 400. In some embodiments, the gastric bypass stent 500 can be installed over a wire (e.g., using fluoroscopy) instead of over the endoscope.

In some embodiments, the gastric bypass stent 500 (or its distal end portion that locks in the anastomosis stent device 400) can be made of a material (e.g., a thermoplastic material) that while heated (e.g., to at least about 60 degrees Celsius) assumes a flexible and malleable state that can conform to the shape of the anastomosis stent device 400 during its delivery to within the anastomosis stent device 400. Then, once distal end portion of the gastric bypass stent 500 is delivered to within the anastomosis stent device 400, and the gastric bypass stent 500 cools to body temperature, it will harden/stiffen and lock in place (i.e., locked with the anastomosis stent device 400).

In some embodiments, the gastric bypass stent 500 can be made of a self-expanding framework and a covering attached to the self-expanding framework. In the depicted embodiment, the gastric bypass stent 500 includes a distal end portion that is coupleable, *in situ,* to interlock with the anastomosis stent device 400 such that the gastric bypass stent 500 proximally extends from the proximal flange 410 to the esophagus 10. The distal end portion of the gastric bypass stent 500 defines a distal opening that is coaxial with the lumen of the anastomosis stent device 400.

The gastric bypass stent 500 includes a proximal end portion 520 defining a proximal opening and a tubular portion 530 extending between the distal end portion 510 and the proximal end portion 520. The gastric bypass stent 500 defines a lumen extending between its proximal opening and distal opening. The lumen of the gastric bypass stent 500 is in fluid communication with the lumen of the anastomosis stent device 400 while the gastric bypass stent 500 is coupled with the anastomosis stent device 400 (as shown). Accordingly, the esophagus 10 is in fluid communication with the jejunum 40 (via the gastric bypass stent 500 and anastomosis stent device 400) so as to bypass the stomach 20 and the duodenum 30.

The gastric bypass stent 500 also includes a collar 540 extending radially outward from the tubular portion 530. The collar 540 is a self-expanding covered stent that snugly engages with the cardia of the stomach 20. The collar 540 can help prevent reflux and stabilize the configuration against proximal or distal migration.

The combined gastric bypass stent 500 and anastomosis stent device 400 is designed to resist migration. For example, the proximal end portion 520 engages with the inner wall of the esophagus 10, the collar 540 engages with the cardia of the stomach 20 (also providing torque resistance), and the anastomosis stent device 400 engages with the walls of the stomach 20 and jejunum 40. Moreover, the anastomosis stent device 400 and the gastric bypass stent 500 are interlocked together that in some iterations can be through magnetized inner rings.

In some embodiments, an anchoring device comprising two rings that are connected to each other (not shown) can be used to anchor the gastric component 500 to the esophagus 10 and the stomach 20. For example, in some embodiments the gastric component 500 can be anchored to the anatomy at the gastroesophageal junction by locking in engagement with the two-ring structure (that is previously anchored, i.e., one ring in the esophagus 10, and one ring in the stomach 20) with one or more clip(s), by endo-suturing, or using mechanical devices or techniques.

Referring also to **FIG. 10****,** optionally, the gastric bypass stent 500 can include a tubular member 550 comprising a flexible tube that is coupleable, *in situ,* with the anastomosis stent device 400 such that the tubular member 550 extends from the distal flange 420 of the anastomosis stent device 400. The tubular member 550 defines a lumen therethrough. The lumen defined by the tubular member 550 is in fluid communication with the lumens of the anastomosis stent device 400 and the gastric bypass stent 500. Using the optional tubular member 550, an addition portion of the jejunum 40 is bypassed. Use of the tubular member 550 can also help to reduce the potential for bile reflux. In some embodiments, the length of the tubular member 550 is between 40 cm and 80 cm.

## Claims

1. A device that is implantable in a gastrointestinal (GI) tract of a patient for reducing caloric uptake and weight of the patient or for improving insulin resistance and diabetes, the device comprising:
a first stent (400) comprising a first self-expanding framework and a first covering attached to the first self-expanding framework, the first self-expanding framework, when expanded, comprising:
a proximal flange (410);
a distal flange (420); and
a waist portion (430) extending between the proximal flange (410) and the distal flange (420), wherein the first stent (400) defines a first lumen extending between the proximal flange (410) and the distal flange (420);
a second stent (500) comprising a second self-expanding framework and a second covering attached to the second self-expanding framework, the second self-expanding framework, when expanded, comprising:
a distal end portion (510) that is coupleable, *in situ,* with the first stent (400) such that the second stent (500) extends from the proximal flange (410), the distal end portion (510) defining a distal opening;
a proximal end portion (520) defining a proximal opening;
a tubular portion (530) extending between the distal end portion (510) and the proximal end portion (520); and
a collar (540) extending radially outward from the proximal end portion of the tubular portion (530), wherein the second stent (500) defines a second lumen extending between the proximal opening and the distal opening, the second lumen being in fluid communication with the first lumen while the second stent (500) is coupled with the first stent (400);
wherein the first stent (400) and the second stent (500) are coupled using magnetism,
wherein the second stent (500) comprises a material that assumes a flexible and malleable state when heated that can conform to the shape of the first stent (400) such that the first stent (400) and the second stent (500) are coupled when the material cools to body temperature, or
wherein the second stent (500) is installed such that the distal end of the second stent (500) is within the first stent (400), such that the first stent (400) and the second stent (500) are coupled when the second stent (500) self-expands within the first stent (400).

2. The device of claim 1, further comprising:
a tubular member (550) comprising a flexible tube (110) that is coupleable, *in situ,* with the first stent (400) such that the flexible tube (110) extends from the distal flange (420), the tubular member (550) defining a third lumen, the third lumen being in fluid communication with the first lumen and the second lumen while the second stent (500) and the flexible tube (110) are coupled with the first stent (400).

3. The device of claim 1, wherein the collar (540) comprises a self-expanding covered member.

4. The device of claim 1, wherein the material that assumes a flexible and malleable state when heated is a thermoplastic material.

5. A system for deploying an implantable device according to any one of the preceding claims, the system comprising:
said implantable device; and
a snare device (200) slidably disposable in the third lumen such that a distal end portion of the snare device can be inserted into the third lumen at the proximal end portion of the flexible tube (110) and extended out of the lateral opening of the flexible tube (110), the snare device (200) comprising:
a sheath (210) defining a fourth lumen; and
an elongate flexible snare member (220) slidably disposed within the fourth lumen, a distal end portion of the snare member (220) configured to emit illumination during use within the GI tract.

6. The device of claim 1, wherein the collar (540) engages with the cardia of the stomach.

## Patentansprüche

1. Vorrichtung, die zur Reduktion der Kalorienaufnahme und des Gewichts des Patienten oder zur Verbesserung der Insulinresistenz und von Diabetes in einen Gastrointestinal-(GI)-Trakt eines Patienten implantierbar ist, wobei die Vorrichtung umfasst:
einen ersten Stent (400), der einen ersten selbstexpandierenden Rahmen und eine erste Abdeckung umfasst, die an dem ersten selbstexpandierenden Rähmen angebracht ist, wobei der erste selbstexpandierende Rahmen, wenn expandiert, umfasst:
einen proximalen Flansch (410);
einen distalen Flansch (420); und
einen Taillenabschnitt (430), der sich zwischen dem proximalen Flansch (410) und dem distalen Flansch (420) erstreckt, wobei der erste Stent (400) ein erstes Lumen definiert, das sich zwischen dem proximalen Flansch (410) und dem distalen Flansch (420) erstreckt;
einen zweiten Stent (500), der einen zweiten selbstexpandierenden Rahmen und eine zweite Abdeckung umfasst, die an dem zweiten selbstexpandierenden Rahmen angebracht ist, wobei der zweite selbstexpandierende Rahmen, wenn expandiert, umfasst:
einen distalen Endabschnitt (510), der, in situ, mit dem ersten Stent (400) gekoppelt werden kann, so dass sich der zweite Stent (500) von dem proximalen Flansch (410) erstreckt, wobei der distale Endabschnitt (510) eine distale Öffnung definiert;
einen proximalen Endabschnitt (520), der eine proximale Öffnung definiert;
einen röhrenförmigen Abschnitt (530), der sich zwischen dem distalen Endabschnitt (510) und dem proximalen Endabschnitt (520) erstreckt; und
einen Kragen (540), der sich von dem proximalen Endabschnitt des röhrenförmigen Abschnitts (530) radial nach außen erstreckt, wobei der zweite Stent (500) ein zweites Lumen definiert, das sich zwischen der proximalen Öffnung und der distalen Öffnung erstreckt, wobei das zweite Lumen mit dem ersten Lumen in Fluidverbindung ist, während der zweite Stent (500) mit dem ersten Stent (400) gekoppelt ist;
wobei der erste Stent (400) und der zweite Stent (500) mittels Magnetismus gekoppelt sind,
wobei der zweite Stent (500) ein Material umfasst, das, wenn es erwärmt wird, einen flexiblen und formbaren Zustand annimmt, der sich an die Form des ersten Stents (400) anpassen kann, so dass der erste Stent (400) und der zweite Stent (500) gekoppelt sind, wenn das Material auf Körpertemperatur abkühlt, oder
wobei der zweite Stent (500) derart eingesetzt wird, dass das distale Ende des zweiten Stents (500) in dem ersten Stent (400) liegt, so dass der erste Stent (400) und der zweite Stent (500) gekoppelt sind, wenn der zweite Stent (500) in dem ersten Stent (400) selbstexpandiert.

2. Vorrichtung nach Anspruch 1, ferner umfassend:
ein röhrenförmiges Element (550), das einen flexiblen Schlauch (110) umfasst, der, in situ, mit dem ersten Stent (400) gekoppelt werden kann, so dass sich der flexible Schlauch (110) von dem distalen Flansch (420) erstreckt, wobei das röhrenförmige Element (550) ein drittes Lumen definiert, wobei das dritte Lumen mit dem ersten Lumen und dem zweiten Lumen in Fluidverbindung ist, während der zweite Stent (500) und der flexible Schlauch (110) mit dem ersten Stent (400) gekoppelt sind.

3. Vorrichtung nach Anspruch 1, wobei der Kragen (540) ein selbstexpandierendes beschichtetes Element umfasst.

4. Vorrichtung nach Anspruch 1, wobei das Material, das, wenn es erwärmt wird, einen flexiblen und formbaren Zustand annimmt, ein thermoplastisches Material ist.

5. System zum Einsetzen einer implantierbaren Vorrichtung nach einem der vorhergehenden Ansprüche, wobei das System umfasst:
die implantierbare Vorrichtung; und
eine Schlingenvorrichtung (200), die verschiebbar in dem dritten Lumen angeordnet ist, so dass ein distaler Endabschnitt der Schlingenvorrichtung in das dritte Lumen an dem proximalen Endabschnitt des flexiblen Schlauchs (110) eingesetzt und aus der lateralen Öffnung des flexiblen Schlauchs (110) herausgefahren werden kann,
wobei die Schlingenvorrichtung (200) umfasst:
eine Hülle (210), die ein viertes Lumen definiert; und
ein längliches flexibles Schlingenelement (220), das verschiebbar in dem vierten Lumen angeordnet ist, wobei ein distaler Endabschnitt des Schlingenelements (220) dazu ausgelegt ist, während der Verwendung in dem GI-Trakt Licht abzugeben.

6. Vorrichtung nach Anspruch 1, wobei der Kragen (540) den Mageneingang in Eingriff nimmt.

## Revendications

1. Dispositif qui est implantable dans un tractus gastro-intestinal (GI) d'un patient pour réduire l'apport calorique et le poids du patient ou pour améliorer la résistance à l'insuline et le diabète, le dispositif comprenant :
un premier stent (400) comprenant une première armature auto-extensible et un premier revêtement fixé à la première armature auto-extensible, la première armature auto-extensible, lorsqu'elle est étendue, comprenant :
une bride proximale (410) ;
une bride distale (420) ; et
une partie taille (430) s'étendant entre la bride proximale (410) et la bride distale (420), dans lequel le premier stent (400) définit une première lumière s'étendant entre la bride proximale (410) et la bride distale (420) ;
un second stent (500) comprenant une seconde armature auto-extensible et un second revêtement fixé à la seconde armature auto-extensible, la seconde armature auto-extensible, lorsqu'elle est étendue, comprenant :
une partie extrémité distale (510) pouvant être accouplée, in situ, avec le premier stent (400) de telle sorte que le second stent (500) s'étende à partir de la bride proximale (410), la partie extrémité distale (510) définissant une ouverture distale ;
une partie extrémité proximale (520) définissant une ouverture proximale ;
une partie tubulaire (530) s'étendant entre la partie extrémité distale (510) et la partie extrémité proximale (520) ; et
un collier (540) s'étendant radialement vers l'extérieur à partir de la partie extrémité proximale de la partie tubulaire (530), dans lequel le second stent (500) définit une deuxième lumière s'étendant entre l'ouverture proximale et l'ouverture distale, la deuxième lumière étant en communication fluidique avec la première lumière pendant que le second stent (500) est accouplé au premier stent (400) ;
dans lequel le premier stent (400) et le second stent (500) sont couplés par magnétisme,
dans lequel le second stent (500) comprend un matériau qui adopte un état flexible et malléable lorsqu'il est chauffé et qui peut épouser la forme du premier stent (400) de telle sorte que le premier stent (400) et le second stent (500) soient accouplés lorsque le matériau refroidit à température corporelle, ou
dans lequel le second stent (500) est installé de telle sorte que l'extrémité distale du second stent (500) se trouve à l'intérieur du premier stent (400), de telle sorte que le premier stent (400) et le second stent (500) soient accouplés lorsque le second stent (500) s'auto-étend à l'intérieur du premier stent (400).

2. Dispositif selon la revendication 1, comprenant en outre :
un élément tubulaire (550) comprenant un tube flexible (110) pouvant être accouplé, in situ, avec le premier stent (400) de telle sorte que le tube flexible (110) s'étende à partir de la bride distale (420), l'élément tubulaire (550) définissant une troisième lumière, la troisième lumière étant en communication fluidique avec la première lumière et la deuxième lumière pendant que le second stent (500) et le tube flexible (110) sont accouplés avec le premier stent (400).

3. Dispositif selon la revendication 1, dans lequel le collier (540) comprend un élément couvert auto-extensible.

4. Dispositif selon la revendication 1, dans lequel le matériau qui adopte un état flexible et malléable lorsqu'il est chauffé est un matériau thermoplastique.

5. Système permettant de déployer un dispositif implantable selon l'une quelconque des revendications précédentes, le système comprenant :
ledit dispositif implantable ; et
un dispositif à anse (200) pouvant être disposé de manière coulissante dans la troisième lumière de telle sorte qu'une partie extrémité distale du dispositif à anse puisse être insérée dans la troisième lumière au niveau de la partie extrémité proximale du tube flexible (110) et s'étendre hors de l'ouverture latérale du tube flexible (110), le dispositif à anse (200) comprenant :
une gaine (210) définissant une quatrième lumière ; et
un élément d'anse flexible allongé (220) disposé de manière coulissante dans la quatrième lumière, une partie extrémité distale de l'élément d'anse (220) étant conçue pour émettre un éclairage pendant l'utilisation dans le tractus gastro-intestinal.

6. Dispositif selon la revendication 1, dans lequel le collier (540) vient en prise avec le cardia de l'estomac.
